# EUROPEAN PATENT SPECIFICATION

(11) **EP 3 967 307 B1**
(45) Date of publication and mention of the grant of the patent: **04.11.2026**
(21) Application number: 20461561.1
(22) Date of filing: 15.09.2020
(51) Int. Cl.: A61K 31/426, A61K 31/444, A61K 31/454, A61K 31/4545, A61K 31/5025, A61K 31/519, A61K 31/551, A61K 38/00, A61K 45/06, A61P 35/00, A61P 35/02, A61K 39/395, C07K 16/28

(54) **PIM KINASE INHIBITORS TO AUGMENT THE EFFICACY OF ANTI-CD20 ANTIBODY-BASED THERAPIES FOR USE IN THE TREATMENT OF HEMATOLOGIC MALIGNANCIES**
PIM-KINASE-INHIBITOREN ZUR VERSTÄRKUNG DER WIRKSAMKEIT VON ANTI-CD20-ANTIKÖRPERBASIERTEN THERAPIEN ZUR VERWENDUNG BEI DER BEHANDLUNG HÄMATOLOGISCHER MALIGNER ERKRANKUNGEN
INHIBITEURS DE KINASE PIM POUR AUGMENTER L'EFFICACITÉ DE THÉRAPIES À BASE D'ANTICORPS ANTI-CD20 POUR UNE UTILISATION DANS LE TRAITEMENT DE MALIGNITÉS HÉMATOLOGIQUES

(43) Date of publication of application: 16.03.2022
(73) Proprietor: Instytut Hematologii i Transfuzjologii, 02-776 Warszawa (PL)
(72) Inventor: JUSZCZYNSKI, Przemyslaw, 05-509 Jozefoslaw (PL); SZYDLOWSKI, Maciej, 05-503 Baszkowka (PL); JABLONSKA, Ewa, 02-776 Warszawa (PL)
(74) Representative: Patpol Kancelaria Patentowa Sp. z o.o.

(56) References cited:
- US-A1- 2007 172 847
- US-A1- 2016 213 652
- US-B2- 9 730 938
- FORSTPOINTNER R. ET AL: "The addition of rituximab to a combination of fludarabine, cyclophosphamide, mitoxantrone (FCM) significantly increases the response rate and prolongs survival as compared with FCM alone in patients with relapsed and refractory follicular and mantle cell lymphomas: results of a prospective randomize", BLOOD, vol. 104, no. 10, 15 November 2004 (2004-11-15), US, pages 3064 - 3071, XP055779883, ISSN: 0006-4971, DOI: 10.1182/blood-2004-04-1323
- HEROLD MICHAEL ET AL: "Rituximab Added to First-Line Mitoxantrone, Chlorambucil, and Prednisolone Chemotherapy Followed by Interferon Maintenance Prolongs Survival in Patients With Advanced Follicular Lymphoma: An East German Study Group Hematology and Oncology Study", JOURNAL OF CLINICAL ONCOLOGY, vol. 25, no. 15, 20 May 2007 (2007-05-20), US, pages 1986 - 1992, XP055779885, ISSN: 0732-183X, DOI: 10.1200/JCO.2006.06.4618
- GARLICH JOSEPH ET AL: "Translational studies of the novel prodrug PI3K/mTOR/PIM1 inhibitor SF1126 in DLBCL", PROCEEDINGS: AACR 102ND ANNUAL MEETING 2011, vol. 71, 15 April 2011 (2011-04-15), pages LB - 383, XP055779891, DOI: 10.1158/1538-7445.AM2011-LB-383
- JIALIN MA ET AL: "A genetic predictive model for precision treatment of diffuse large B-cell lymphoma with early progression", BIOMARKER RESEARCH, BIOMED CENTRAL LTD, LONDON, UK, vol. 8, no. 1, 26 August 2020 (2020-08-26), pages 1 - 11, XP021280865, DOI: 10.1186/S40364-020-00214-3
- JACOBS MARC D. ET AL: "Pim-1 Ligand-bound Structures Reveal the Mechanism of Serine/Threonine Kinase Inhibition by LY294002", JOURNAL OF BIOLOGICAL CHEMISTRY, vol. 280, no. 14, April 2005 (2005-04-01), pages 13728 - 13734, XP055780085, ISSN: 0021-9258, DOI: 10.1074/jbc.M413155200
- WAN XIAOBO ET AL: "A New Target for an Old Drug: Identifying Mitoxantrone as a Nanomolar Inhibitor of PIM1 Kinase via Kinome-Wide Selectivity Modeling", JOURNAL OF MEDICINAL CHEMISTRY, vol. 56, no. 6, 15 March 2013 (2013-03-15), US, pages 2619 - 2629, XP055780088, ISSN: 0022-2623, DOI: 10.1021/jm400045y
- WANG WEI ET AL: "Long Noncoding RNA KCNQ1OT1 Confers Gliomas Resistance to Temozolomide and Enhances Cell Growth by Retrieving PIM1 From miR-761", CELLULAR AND MOLECULAR NEUROBIOLOGY, SPRINGER NEW YORK, US, vol. 42, no. 3, 8 September 2020 (2020-09-08), pages 695 - 708, XP037706792, ISSN: 0272-4340, [retrieved on 20200908], DOI: 10.1007/S10571-020-00958-4

## Description

### FIELD OF THE INVENTION

The present invention relates certain PIM kinase inhibitors to augment the efficacy of anti-CD20 antibody-based therapies for use in the treatment of hematologic malignancies. Moreover, the invention discloses combination therapy pharmaceutical composition comprising said PIM inhibitors and anti-CD20 antibody and said combination therapy for use in the treatment of hematologic malignancy.

### BACKGROUND OF THE INVENTION

Diffuse large B-cell non-Hodgkin lymphoma (DLBCL) is the most common aggressive B-cell lymphoma in adults. DLBCL incidence increases with age and is 3-4/100,000/year in Europe and approximately 4.68 cases per 100,000 per year in the USA. An estimated 10,000 deaths result from DLBCL annually in the USA (1).

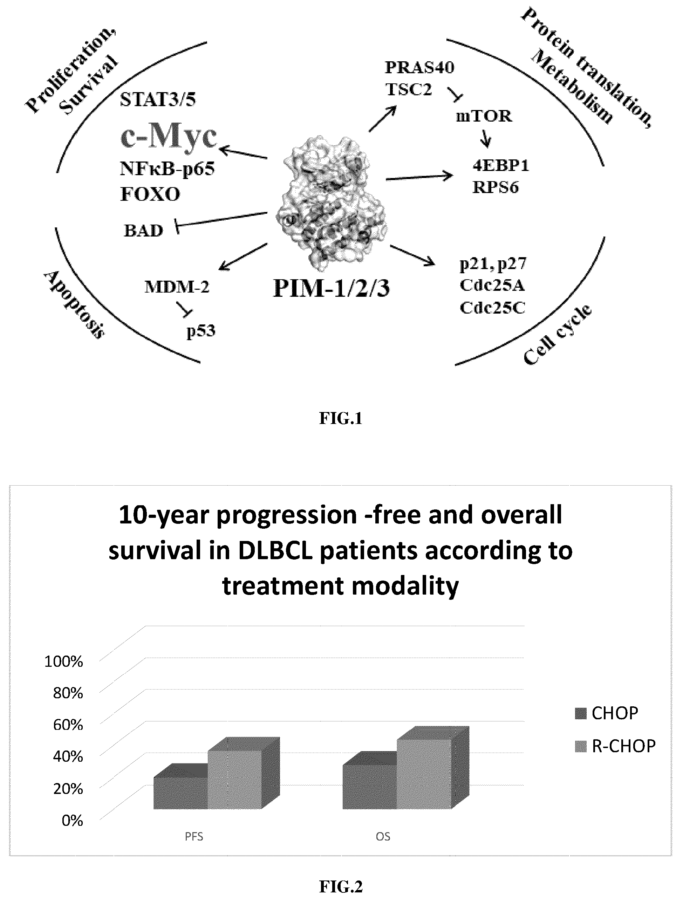

DLBCL exhibits highly heterogenous clinical behavior and a complex molecular background, with multiple low-frequency mutations, somatic copy number alterations, and structural variants (2). The heterogeneity of DLBCL is reflected in transcriptionally defined subtypes that provide insights into disease pathogenesis, determine patient's risk of treatment failure, and are candidate treatment targets. MYC-IgH translocations are the third most frequent rearrangement in DLBCL, occurring in 5-15% of patients. MYC protein expression is detected in a much higher proportion of DLBCL (30%-50%), and usually it is associated with suboptimal responses to frontline treatment and inferior prognosis (3,4). MYC encodes a leucine zipper transcription factor that modulates a broad spectrum of cellular genes responsible for enhancing cell proliferation, cellular metabolism, growth, angiogenesis, metastasis, genomic instability, stem cell self-renewal and reduced differentiation (5). In mouse models, MYC induces lymphomas, but requires cooperation with other lesions. For example, simultaneous overexpression of PIM1 kinase stabilizes MYC expression and highly accelerates MYC - induced lymphomagenesis (6). The family of PIM serine/threonine kinases comprises three highly conserved oncogenes (PIM1, -2, and -3), overexpressed in various solid cancers and hematological malignancies. PIMs lack a regulatory domain; thus, they become fully activated once transcribed (7). PIMs have been shown to promote oncogenesis by modulating the activity of proteins involved in regulation of translation, transcription, cell cycle and survival (8-12) (Figure 1).

Regardless of molecular background and biological/genetic prognostic factors, the current approach to frontline therapy for DLBCL patients is uniform and consists of cyclophosphamide, doxorubicin, vincristine and prednisone (CHOP) based chemotherapy combined with anti- CD20 antibody rituximab. Introduction of rituximab to CHOP therapy (R-CHOP) almost two decades ago significantly improved the treatment outcomes of DLBCL patients. Results of the long-term, multicenter study (13) clearly indicated longer progression-free and overall survival time in DLBCL patients treated with R-CHOP than CHOP (Figure 2).

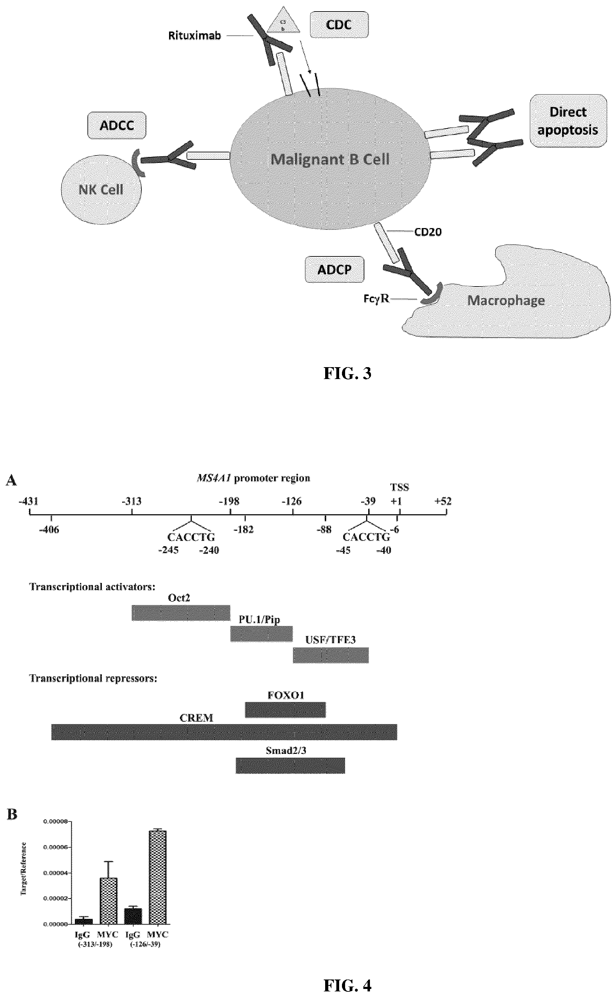

Encoded by MS4A1 gene, cell surface protein CD20 is a 33-kDa protein, which is expressed almost exclusively on B cells during different stages of their development, starting at pre-B-cell stage, but is lost as cells differentiate to plasma cells. CD20 is a tetraspanin, with both the C- terminal and N-terminal tails within the cell, 4 transmembrane domains and two extracellular loops. CD20 is not shed or internalized, making it an ideal antigen for targeting with therapeutic antibodies. After association with the cognate antigen, anti-CD20 antibodies trigger three different effector mechanisms: complement-dependent cytotoxicity (CDC), antibody-dependent cellular cytotoxicity (ADCC) mediated by cells displaying Fc receptors (FcRs), such as FcRIIIa-expressing NK cells and macrophages, and direct antitumor effect via either apoptosis or other cell death pathways (Figure 3).

Importantly, CD20 expression levels determine responsiveness of lymphomas to rituximab (14-17). Patients with high pre-treatment CD20 surface expression respond better to standard doses of rituximab and have significantly better prognosis than those with decreased CD20 abundance.

Moreover, what should be emphasized, a substantial fraction of patients with B-NHL fails first-line, rituximab-based therapy, and many patients with relapse acquire resistance to rituximab. Studies performed so far emphasize that reduction of CD20 antigen expression is an important mechanism of acquired resistance to rituximab and highlight potential therapies to re-sensitize resistant cells by upregulating CD20 levels in B-NHL cells (18, 19). However, none of these therapies have been applied in the treatment of these patients to date.

Since reduced CD20 levels predict suboptimal rituximab responses, it is important to understand mechanisms modulating CD20 expression. It was reported that targeted therapies blocking B-cell receptor signaling antagonize CD20 expression and decrease rituximab-induced CDC (20-22), mandating caution for simultaneous/synchronous use of potentially antagonistic drugs. Albeit knowledge of drugs and drug-related mechanisms decreasing CD20 expression is important to avoid pharmacologic antagonisms, identification of drugs working synergistically with anti-CD20 antibodies through boosting CD20 expression levels would be more interesting to clinicians and patients.

In the prior art, a method for predicting the responsiveness of a patient to a treatment with an anti-CD20 antibody is disclosed. For example, European patent publication EP3114233B1 describes a method that includes measuring the level of glyceraldehyde-3-phosphate de-hydrogenase (GAPDH) expression in B-cells obtained from a patient. Moreover, patients with high GAPDH expression have a higher CD20 expression and a better response to anti-CD20 based therapy.

Subsequent patent document no. EP1112084B1 discloses to the use of anti-CD20 antibodies or fragments thereof in the treatment of B-cell lymphomas, particularly the use of such antibodies and fragments in combined therapeutic regimens. In particular, it has been found that treatment with anti-CD20 antibody provides a beneficial synergistic effect when administered in combination with cytokines, radiotherapy, myeloablative therapy, or chemotherapy.

There are also known some molecules that affect the modulation of the expression level of CD20 antigen on the cell surface are also known in the prior art.

For example, patent document no. EP2573176B1 discloses novel specific oligonucleotide sequences, showing pronounced therapeutic effects either alone or in combination with other treatments. There are provided some compounds and methods for the treatment of cancer, wherein an isolated oligonucleotide is used either alone; to increase apoptosis, to activate NK-cells, to up-regulate the expression of one or more of the cell surface markers CD20, CD23, CD25, CD40, CD54, CD69, CD80, and CD86; or in combination with an anti-tumour therapy, most preferably said anti-tumour treatment is an immunological treatment and comprises the administration of an antibody to the patient, selected from but not limited to, Rituximab (Rituxan^{®}, MabThera^{®}), Lumiliximab, Alentuzumab (Campath^{®}, MabCampath^{®}, Bevacizumab (Avastin^{®}), and Trastuzumab (Herceptin^{®}).

International patent application WO2009/108912Al, in turn, provides chemical compounds having certain biological activities that include, but are not limited to, inhibiting cell proliferation, inhibiting angiogenesis, and modulating protein kinase activity. These molecules can modulate casein kinase 2 (CK2) activity, PIM kinase activity, and/or Fms-like tyrosine kinase 3 (Flt3) activity and thus affect biological functions that include but are not limited to, inhibiting gamma phosphate transfer from ATP to a protein or peptide substrate, inhibiting angiogenesis, inhibiting cell proliferation and inducing cell apoptosis. Compounds of the invention are useful when used in combination with antibodies, i.e. rituximab, ticilimumab, trastuzimab and the like.

Moreover, international patent application WO2017/044730A1 discloses some salt forms of the PIM kinase inhibitor as well describes methods of preparation thereof, and intermediates useful in such methods. The compounds disclosed herein are useful in the treatment of PIM kinase-related diseases such as cancer. PIM kinase associated diseases that can be treated according to the invention described herein include cancer, including, in particular, cancers in which PIM kinases are upregulated or an oncogene, e.g., Myc or BCL2, is activated. The PIM inhibitor can further be used in combination with other methods of treating cancers, for example the salts of the invention can be administered in combination with one or more anti-cancer drugs, such as a chemotherapeutics. Example chemotherapeutics include any of, i.e. rituximab.

Additionally, international patent application WO2019/040724A1 discloses a new class of small-molecules inhibitors of Myc, having an oxadiazole-oxadiazolone structure, and their use as therapeutics for the treatment of cancer and other diseases. According to this publication a number of suitable anticancer agents are contemplated for use in the methods disclosed herein. In certain embodiments, anticancer agents comprise agents that induce or stimulate apoptosis, i.e. antibodies (e.g., herceptin, rituxan, zevalin, and avastin).

It was also demonstrated that some inhibitors of SRC kinases impair antitumor activity of anti-CD20 monoclonal antibodies (23). More specifically, a transcriptional profiling of tumor cells incubated with SFKs inhibitors revealed strong downregulation of MS4A1 gene encoding CD20 antigen. In a panel of primary and established B-cell tumors the authors observed that SFKs inhibitors strongly affect CD20 expression at the transcriptional level, leading to inhibition of anti-CD20 mAbs binding and increased resistance of tumor cells to complement-dependent cytotoxicity.

PIM kinases are also known in the prior art to promote cell cycle progression and to inhibit apoptosis leading to tumorigenesis. However, the precise mechanisms of this process remain unclear. As it is known, PIM kinases have the ability to suppress *p27kip1* transcription through phosphorylation and inactivation of forkhead transcription factors, including FOXO1 and FOXO3. PIM-1 inactivation of FOXO1 would have deleterious effects on B cell fate determination as FOXO1 is an integral member of a global network of transcription factors, including E2A and EBF1. (24, 25). Activated FOXO1 promotes resistance of non-Hodgkin lymphomas to anti-CD20-based therapy. Authors found that - CD20 transcription is negatively regulated by FOXO1 in NHL cell lines and in human lymphoma specimens carrying activating mutations of *FOXO1.* Accordingly, pharmacological inhibition of FOXO1 activity in primary samples upregulated surface CD20 levels (22). Therefore, PIM kinases do not seem be promising entities positively regulating -CD20.

Additionally, studies in murine and human models of B-cell lymphomas suggested that MYC may act to suppress CD20 expression in B lymphocytes (26, 27). However, inactivation of MYC by its direct targeting is challenging and most probably would be not achievable in a clinical setting. First, MYC is hardly druggable target, as it lacks a specific active/docking site for small molecules. Second, due to its predominantly nuclear localization, targeting MYC with specific monoclonal antibodies is technically not feasible. Third, MYC plays broad end essential role in many normal developing and mature tissues and its direct and non-selective targeting leads to severe toxicities. Mice with germ-line cMYC knockout are not viable and die during embryonic development (between day E9.5 and 10.5), whereas hematopoietic cell - specific MYC inactivation profoundly disturbs normal haematopoiesis, with severe thrombocytosis-anemia-leukopenia syndrome. For these reasons, direct MYC targeting is not clinically applicable. Instead, indirect MYC targeting in tumor cells specifically reliant on this oncogene appears a more rational, reasonable and potentially clinically useful strategy. Moreover, the above publications describe experiments relating to Burkitt's lymphoma (BL), not to diffuse large B-cell lymphoma (DLBCL), as in the present invention. Although, diffuse large B-cell lymphomas (DLBCLs) and Burkitt lymphoma (BL) account for the majority of aggressive lymphomas in adults and children, the clinical course of these two malignancies significantly differs, and despite multiple studies, the biological grounds for this remain unclear. Burkitt's lymphoma (BL) defines a distinct group of B-cell malignancies that are primarily found in the germinal centres (secondary follicles) of the peripheral lymph nodes. The World Health Organization (WHO) defines the BL as an aggressive non-Hodgkin lymphoma (NHL) originating from the mature stage B lymphocytes. Distinguishing between BL and DLBCL is critical, as the two diseases require different management. DLBCLs exhibit marked biological heterogeneity and variable clinical presentation and clinical course. Conversely, BL is genetically relatively homogeneous but associated with variable clinicopathological features. In addition, there are some suggestions that inhibitors of kinases may interfere with the anti-tumor activity of anti-CD20 antibodies. Moreover, it is well known that MYC is regulated by many kinases and CD20 has many regulatory mechanisms. Therefore, there is still great need to provide the method according to which it would be possible efficiently increase CD20 antigen.

Since regulation of CD20 expression, more particularly, upregulating of CD20 levels in B-NHL cells seems to be highly desirable it is a great need of the new methods and molecules that would increase the numbers of CD20 proteins in such cells increasing their sensitivity to the therapy based on anti-CD20 antibodies. Moreover, there is also widespread demand for pharmaceutical product, such as pharmaceutical composition or formulation, which can be effectively and safely used in the treatment of diseases associated with downregulation of CD20 levels in B-NHL cells.

Inventors of the present invention surprisingly found that certain PIM kinase inhibitors not only may effectively impact CD20 regulatory mechanism but also provide synergistic effect when simultaneously used with anti-CD20 antibodies in the treatment of hematological malignancies None of earlier disclosures provides any indication or even suggestion that PIM inhibitors might be successfully used as CD20 regulators, and especially, that they can be effectively used in combination therapy with anti-CD20 antibody for use as a highly effective method of treating individuals suffering from hematological malignancies.

As already emphasized, although the relationships between MYC and CD20, and between MYC and PIM are known, obtaining such a significant synergistic effect of use of PIM kinase inhibitors to augment the efficacy of anti-CD20 antibody-based therapies was extremely surprising and unexpected for the inventors of the present invention. Especially, that in cellular signalling pathways, compensation of signal transduction is often observed. Moreover, a person skilled in the art would also not feel motivated to conduct research in this direction, as the authors themselves indicated that Western blot analysis showed moderately increased protein expression for several B-cell antigens (CD20, CD79a, BLNK, PAX5) (27), and not, as in the present invention, a significant and unexpected increase in the expression level of CD20 antigen.

In summary, without being bound to any theory, these publications confirmed the existence of a potential, second and opposite mechanism, because inhibition of PIM kinases causes a decrease in FOXO1 phosphorylation and thus its activation, and activated FOXO1 enters the nucleus and inhibits the expression of CD20. There are probably more such mechanisms but they have not yet been characterized, so it was impossible to predict the consequences of PIM inhibition on CD20 expression, because these two examples (FOXO-CD20 and MYC-CD20) show opposite effects. So the key issue was to present how inhibition of PIMs affects CD20, and the potential mechanism is probably less important.

### SUMMARY OF THE INVENTION

The present invention is defined in the appended claims. Any embodiments which do not fall under the scope of the claims are not the subject of the present invention.

The references to the methods of treatment of this description are to be interpreted as references to compounds, pharmaceutical compositions and medicaments of the present invention for use in a method for treatment of the human (or animal) body by therapy.

The present invention based on the founding that targeting PIM kinases increases CD20 expression and its cell surface abundance.

The invention provides a PIM inhibitor selected from the group comprising SGI-1776, PIM447 and SEL24/MEN1703 for use in the treatment of hematological malignancies in combination therapy with anti-CD20 antibody. It further provides a combination therapy pharmaceutical composition comprising an effective amounts of said PIM inhibitor and an anti-CD20 antibody as active ingredients, and pharmaceutically acceptable excipients and said composition for use in the treatment of hematological malignancy.

Preferably, hematological malignancy includes, but is not limited to, diffuse large B-cell lymphoma, high-grade B-cell lymphoma with MYC and BCL2 and/or BCL6 rearrangements, chronic lymphocytic leukemia/lymphocytic lymphoma, mantle cell lymphoma, Burkitt lymphoma, follicular lymphoma, marginal zone lymphoma. More preferably, said hematological malignancy is diffuse large B-cell lymphoma or Burkitt lymphoma.

The PIM inhibitor is selective for one or more PIM kinase isoforms. The PIM inhibitor of the present invention is selected from SGI-1776, PIM447 and SEL24/MEN1703. Other PIM kinase inhibitors not encompassed in the present invention are SMI-4a, SMI-16a, TP-3654, AZD1208, INCB053914, CX-6258. The PIM kinase inhibitor is an inhibitor of MYC expression.

Moreover, the present invention provides PIM inhibitor for use in the treatment of hematological malignancies in combination therapy with anti-CD20 antibody, wherein the treatment comprises administering to the patient a therapeutically effective amount of a PIM kinase inhibitor and a therapeutically effective amount of anti-CD20 antibody. Preferably, the patient is one in whom MYC is overexpressed or the patient is one in whom PIM kinase is overexpressed.

In one preferred embodiment, the administration of the PIM kinase inhibitor and anti-CD20 antibody is performed simultaneously, more specifically concurrently.

In further preferred embodiment, the PIM kinase inhibitor and anti-CD20 antibody are administered sequentially. Preferably, the administration of the first dosage of PIM kinase inhibitor is performed before administration of the first dosage anti-CD20 antibody, more preferably by at least 12 hours before, even more preferably 12 or 24 or 48 hours before, most preferably 24 hours before.

In yet preferred embodiment, administration of the PIM kinase inhibitor and anti-CD20 antibody is performed in therapeutic cycles. Preferably, said therapeutic cycles include dosing of PIM kinase inhibitor for 14 days from day 1 and administration of anti-CD20 antibody from day 2, with 24 hours delay to administration of PIM kinase inhibitor in cycles, every 21 days.

However, other dosage regimens are not excluded, and it is understood by one of skill in the art that the dosage of a given substance, in this case the inhibitor or the antibody, depends on its specific pharmacokinetics.

In yet another preferred embodiment, the administration of the PIM inhibitor and the anti-CD20 antibody is performed simultaneously, more specifically concurrently, with additional therapy, preferably with anthracycline- containing chemotherapy, more preferably with CHOP immunochemotherapy.

The invention also provides also pharmaceutical composition for combination therapy comprising as an active ingredients an effective amount of PIM inhibitor, and an effective amount of anti-CD20 antibody and pharmaceutically acceptable excipients. Preferably, the active ingredients of such composition are formulated in separate dosage formulations. More preferably, separate dosage formulations are intended to be administered simultaneously, more specifically concurrently, under different dosage regimes.

In the preferred embodiment, the combination therapy pharmaceutical composition according to present invention is for use in the treatment of hematologic malignancy.

### DEFINITIONS

The term "synergistic effect" is an effect arising between two or more agents, drugs or substances that produces an effect greater than the sum of their individual effects. Effects of drug combinations are herein formally assessed by calculating combination indices (CI) according to Chou-Talalay method. For additive effect (CI = 1), synergism (CI < 1), and antagonism (CI > 1).

For the terms "for example" and "such as" and grammatical equivalences thereof, the phrase "and without limitation" is understood to follow unless explicitly stated otherwise. As used herein, the term "about" is meant to account for variations due to experimental error. All measurements reported herein are understood to be modified by the term "about", whether or not the term is explicitly used, unless explicitly stated otherwise. As used herein, the singular forms "a," "an," and "the" include plural referents unless the context clearly indicates otherwise.

As used herein, the term "hematological malignancy" includes lymphoma, leukemia, myeloma, or lymphoid malignancies, as well as cancers of the spleen and lymph nodes. Exemplary lymphomas include chronic lymphocytic leukemia/small lymphocytic lymphoma, Monoclonal B-cell lymphocytosis, B-cell prolymphocytic leukemia, Splenic marginal zone lymphoma, Hairy cell leukemia, Splenic B-cell lymphomalleukemia, unclassifiable, Splenic diffuse red pulp small B-cell lymphoma, Hairy cell leukemia-variant, Lymphoplasmacytic lymphoma, Waldenström macroglobulinemia, Monoclonal gammopathy of undetermined significance (MGUS), IgM, µ heavy-chain disease, γ heavy-chain disease, α heavy-chain disease, monoclonal gammopathy of undetermined significance (MGUS), IgG/A plasma cell myeloma, Solitary plasmacytoma of bone, Extraosseous plasmacytoma, Monoclonal immunoglobulin deposition diseases, Extranodal marginal zone lymphoma of mucosa-associated lymphoid tissue (MALT lymphoma), Nodal marginal zone lymphoma, Pediatric nodal marginal zone lymphoma, Follicular lymphoma, In situ follicular neoplasia, Duodenal-type follicular lymphoma, Pediatric-type follicular lymphoma, Large B-cell lymphoma with IRF4 rearrangement, Primary cutaneous follicle center lymphoma, Mantle cell lymphoma, In situ mantle cell neoplasia, Diffuse large B-cell lymphoma (DLBCL), NOS , Germinal center B-cell type DLBCL, Activated B-cell type DLBCL, T-cell/histiocyte-rich large B-cell lymphoma, Primary DLBCL of the central nervous system (CNS), Primary cutaneous DLBCL, leg type , EBV+ DLBCL, NOS , EBV+mucocutaneous ulcer, DLBCL associated with chronic inflammation, Lymphomatoid granulomatosis, Primary mediastinal (thymic) large B-cell lymphoma, Intravascular large B-cell lymphoma, ALK+ large B-cell lymphoma, Plasmablastic lymphoma, Primary effusion lymphoma, HHV8+DLBCL, NOS, Burkitt lymphoma, Burkitt-like lymphoma with 11q aberration, High-grade B-cell lymphoma, with MYC and BCL2 and/or BCL6 rearrangements, High-grade B-cell lymphoma, NOS , B-cell lymphoma, unclassifiable, with features intermediate between DLBCL and classical Hodgkin lymphoma, Nodular lymphocyte predominant Hodgkin lymphoma, Nodular sclerosis classical Hodgkin lymphoma, Lymphocyte-rich classical Hodgkin lymphoma, Mixed cellularity classical Hodgkin lymphoma, Lymphocyte-depleted classical Hodgkin lymphoma, Posttransplant lymphoproliferative disorders (PTLD), Plasmacytic hyperplasia PTLD, Infectious mononucleosis PTLD, Florid follicular hyperplasia PTLD, Polymorphic PTLD, Monomorphic PTLD (B- and T-/NK-cell types), Classical Hodgkin lymphoma PTLD. Those skilled in the art should be clear that these malignant diseases often have different names due to changes in the classification system, and patients with lymphomas classified under different names can also benefit from the combined treatment scheme of the present invention.

The term "anti-CD20 antibody" refers to an antibody directed against the CD20 antigen. The CD20 antigen is expressed on B lymphocytes. Examples of anti-CD20 antibodies include but are not limited to rituximab, ibritumomab, ofatumumab, ocrelizumab, PRO131921, veltuzumab, AME-133v, tositumomab, obinutuzumab (GA-101).

As used herein, the term "inhibitor" is meant to describe a compound that blocks or reduces an activity of an enzyme or system of enzymes, receptors, or other pharmacological target. An inhibitor can act with competitive, uncompetitive, or noncompetitive inhibition. An inhibitor can bind reversibly or irreversibly, and therefore the term includes compounds that are suicide substrates of an enzyme. An inhibitor can modify one or more sites on or near the active site of the enzyme, or it can cause a conformational change elsewhere on the enzyme. The term inhibitor is used more broadly herein than scientific literature so as to also encompass other classes of pharmacologically or therapeutically useful agents, such as agonists, antagonists, stimulants, co-factors, and the like.

The term "PIM inhibitor" refers to any molecule that can interact with PIM kinase or a PIM-related polypeptide and inhibit the catalytic activity and/or non-catalytic function of PIM kinases. Compounds within the scope of the invention can be naturally occurring or chemically synthesized. The term is also intended to include pharmaceutically acceptable salts of the compounds.

As used herein, the term "knockout" refers to an animal or cells therefrom, in which the insertion of a transgene disrupts an endogenous gene in the animal or cell therefrom. This disruption can essentially eliminate PIM in the animal or cell.

A "therapeutically effective amount" of a compound with respect to the subject method of treatment, refers to an amount of the compound(s) in a preparation which, when administered as part of a desired dosage regimen (to a patient, e.g., a human) alleviates a symptom, ameliorates a condition, or slows the onset of disease conditions according to clinically acceptable standards for the disorder or condition to be treated or the cosmetic purpose, e.g., at a reasonable benefit/risk ratio applicable to any medical treatment.

The term "treated, "treating" or "treatment" includes the diminishment or alleviation of at least one symptom associated or caused by the state, disorder or disease being treated. For example, treatment can be diminishment of one or several symptoms of a disorder or complete eradication of a disorder.

The term "resistant" as used herein is meant to refer to cancers that do not respond to drug treatment. The cancer may be resistant at the start of treatment, or it may become resistant during treatment.

A "patient" as used herein refers to a mammal. Preferably, the mammal may be a human. In some embodiments, the patient is one in whom *Myc* is overexpressed.

The term "sequentially" or "sequential administration" as used herein is intended to mean that one of active agent, PIM-kinase inhibitor, is administered before second subsequently administered active agent, anti-CD20 antibody, or that a first active agent is released into the bloodstream at a faster rate than second active agent. In various embodiments, the first active agent is administered 12 hours, 24 hours, 48 hours or 72 hours before the subsequently administered active agent.

The term "simultaneously" as used herein is intended to mean that one of active agent, PIM-kinase inhibitor, is administered at the same time as the second active agent, anti-CD20 antibody. A particular example of "simultaneous administration" is "concurrent administration". This term as used herein is intended to mean that one of active agent, PIM-kinase inhibitor, is administered exactly at the same time as the second active agent, anti-CD20 antibody.

### BRIEF DESCRIPTION OF THE DRAWINGS

The invention will be described in closer detail in the following description, non-limiting examples and claims, with reference to the attached drawings, in which:
FIG. 1. presents pathogenetic mechanisms associated with activity of PIM kinases. Oncogenic PIM kinases modulate of activity of molecules involved in key cellular processes, including proliferation, metabolism, protein translation, cell cycle and apoptosis;
FIG. 2. shows that introduction of rituximab improves outcome of CHOP-treated DLBCL patients. Progression-free and overall survival of DLBCL patients treated with CHOP-chemotherapy versus CHOP-Rituximab immunochemotherapy. Source data from: Coiffier et al, Blood. 2010 Sep 23; 116(12): 2040-2045;
FIG. 3. presents mechanisms of action of rituximab. Rituximab is a monoclonal antibody directed against CD20 antigen, which is expressed on the surface of B cells. The recognition of rituximab through the FcγR receptor mediates the lysis of B cells by NK cells (Antibody-Dependent Cell-Mediated Cytotoxicity, ADCC), or phagocytosis by macrophages (Antibody-Dependent Cellular Phagocytosis, ADCP). Rituximab also induces the classical activation of the of complement (CDC). The activation of complement cascade forms a transmembrane channel, leading to cell lysis. Rituximab may also directly elicit apoptosis of CD20-positive B cells;
FIG. 4. presents that MYC binds to promoter of MS4A1 gene, coding for CD20 protein. (A) Known transcriptional activators and repressors of MS4A1. Upper diagram illustrates MS4A1 promoter region. Annotated positions refer to cis regulatory elements localized upstream transcription start site (TSS, +1), harboring binding sites for transcription factors indicated below. Two non-canonical E-boxes (CACCTG) were identified within the proximal MS4A1 promoter (-245/-240 and -45/-40); (B) Chromatin immunopreipitation experiment showing MYC binding to fragments -313/-198 and - 126/-39 of MS4A1 promoter in DHL4 cells. Error bars represent the standard deviation (SD) of three independent replicates in a representative experiment;

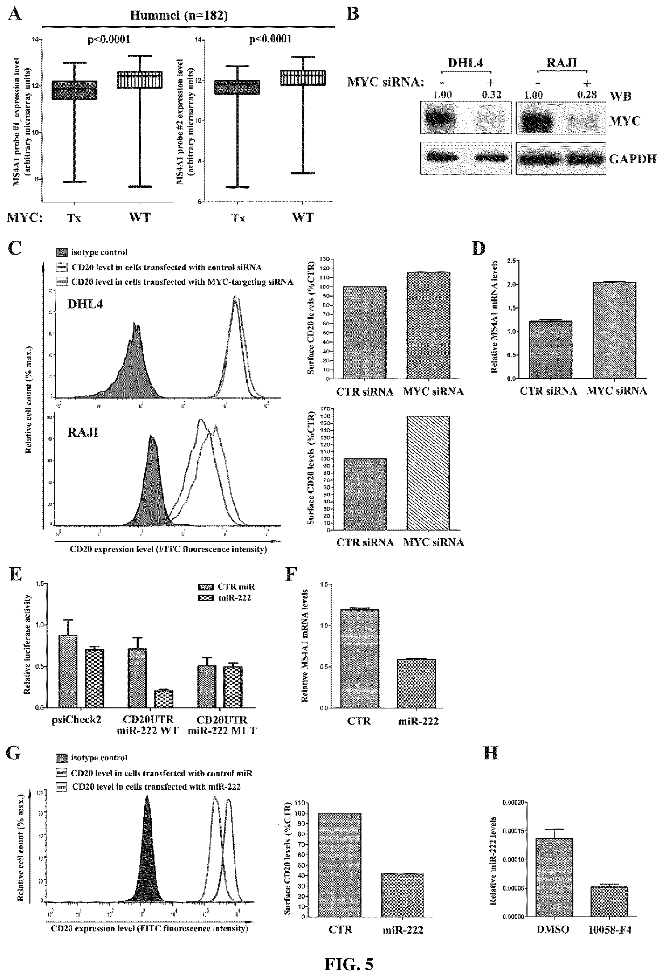

FIG. 5. shows that MYC represses CD20 expression in B-NHL cells. (A) Higher MS4A1 mRNA levels in DLBCL and BL patients with MYC translocation (Tx), compared to patients with wild-type MYC locus (WT). MS4A1 gene expression data from publicly available microarray dataset (GEO accession number: GSE4475) for two independent MS4A1 probes are shown. P values were calculated using Mann-Whitney test;
   (B) RNAi-mediated MYC protein knock-down in DHL4 and RAJI cells. Cells were electroporated with a non-targeting control siRNA (-) or with a siRNA targeting MYC (+) and cultured for 48 hours. Thereafter, cells were lysed and MYC expression was assessed by immunoblotting. GAPDH served as a loading control;
   (C) MYC knock-down increases surface CD20 expression. DHL4 and RAJI cells were electroporated with a nontargeting control siRNA (CTR) or with a siRNA targeting MYC as in panel (B). CD20 expression was assessed by flow cytometry. Bars adjacent to histograms represent median fluorescence intensity (MFI) of CD20 calculated as a percent control siRNA-transfected cells (CTR);
   (D) MYC silencing elevates MS4A1 mRNA levels. DHL4 cells were electroporated with a nontargeting control siRNA (CTR) or with a siRNA targeting MYC. Thereafter, cells were cultured for 48 hours and MS4A1 mRNA abundance was assessed by qPCR using 2^{-ΔΔCT} method;
   (E) MiR-222 targets CD20. HEK293T cells were cotransfected with psiCheck-2 vector containing wild-type (WT) or mutated (MUT) 3'-UTR sequence of MS4A1 and miR-222 mimic or a nontargeting control mimic (CTR miR). A dual luciferase assay was performed after 24 hours. Error bars represent the standard deviation (SD) of three independent replicates in a representative experiment;
   (F) MiR-222 decreases MS4A1 mRNA levels in DLBCL cells. DHL4 cells were transfected with a nontargeting control mimic (CTR) or miR-222 mimic. After 48 hours MS4A1 mRNA levels were determined by qPCR using 2^{-ΔΔCT} method. Error bars represent the standard deviation (SD) of three independent replicates in a representative experiment;
   (G) MiR-222 overexpression downregulates surface CD20 abundance in DLBCL cells. DHL4 cells were transfected with a nontargeting control mimic (CTR) or miR-222 mimic. After 48 hours, surface CD20 expression was assessed by flow cytometry. Bars adjacent to histogram represent median fluorescence intensity (MFI) of CD20 calculated as a percent control mimic-transfected cells (CTR);
   (H) MYC regulates MiR-222 expression in DLBCL cells. DHL4 cells were treated with DMSO alone or MYC inhibitor, 10058-F4 (50µM), for 24 hours. Afterwards, miR-222 expression was assessed by qPCR using 2^{-ΔΔCT} method;

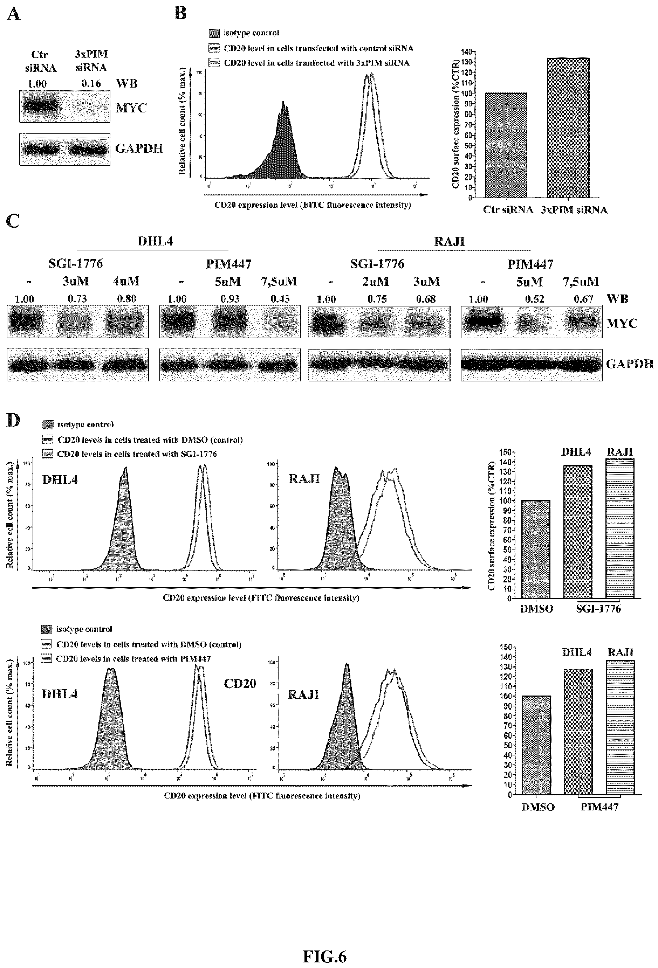

FIG. 6. presents that PIM kinase inhibition in DLBCL and BL cells downregulates MYC protein and elevates CD20 surface abundance. (A) PIM1, -2 and -3 protein knock-down decreases MYC abundance in DLBCL cells. DHL4 cells were electroporated with a non-targeting siRNA or with siRNA cocktail targeting all three PIM (3xPIM siRNA) kinases. Thereafter, cells were cultured for 48h and MYC expression was assessed by immunoblotting. GAPDH served as a loading control;
   (B) Induction of CD20 surface levels in DHL4 cells after silencing PIM kinases. Cells were electroporated with siRNAs as in (A). CD20 surface abundance was assessed 96h after electroporation with siRNAs by flow cytometry. Bars adjacent to histogram represent median fluorescence intensity (MFI) of CD20 calculated as a percent control siRNA-transfected cells (CTR);
   (C) Chemical PIM kinase inhibition downregulates MYC protein in DLBCL and BL cells. DHL4 (DLBCL) and RAJI (BL) cells were treated for 24 hours with DMSO alone or SGI-1776, or PIM447 at indicated concentrations. Afterwards, MYC expression was assessed by immunoblotting. GAPDH served as a loading control;
   (D) Pan-PIM inhibitors elevate surface CD20 expression in DLBCL and BL cells. DHL4 (DLBCL) cells were treated with DMSO alone, 3µM SGI-1776 for 48h or 5µM PIM447 for 72h. RAJI (BL) cells were incubated with DMSO, 2µM SGI-1776 for 48h or 5µM PIM447 for 72h. Afterwards, CD20 surface abundance was measured by flow cytometry. Bars adjacent to histograms represent median fluorescence intensity (MFI) of CD20 calculated as a percent control DMSO-treated cells (CTR);

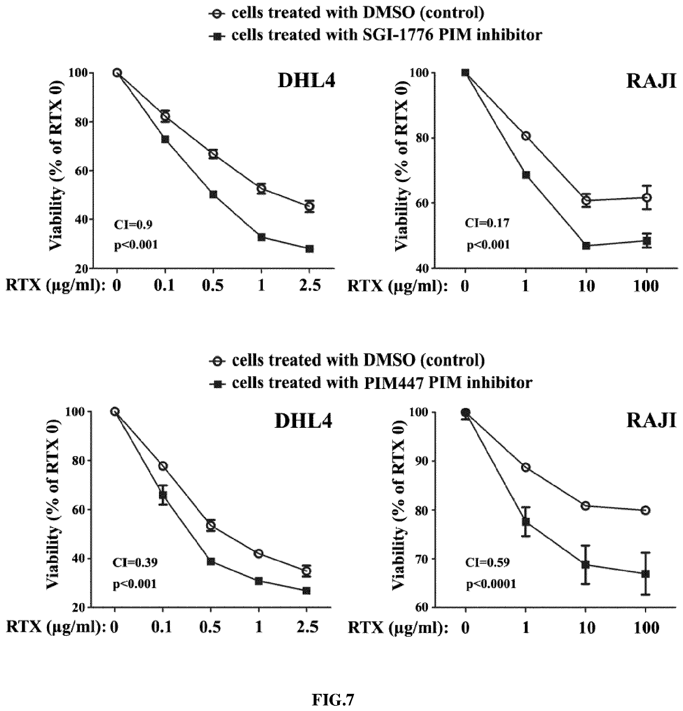

FIG. 7. presents that PIM inhibitors and Rituximab synergistically induce DLBCL and BL cell death via CDC. Graphs demonstrate increased apoptotic response (i.e. decreased cell viability) to rituximab in pan-PIM inhibitor-pretreated DHL4 and RAJI cells. Cells were incubated with DMSO, SGI-776 or PIM447 as described in Figure 4D. Thereafter, cells were exposed to rituximab at indicated concentrations (0.1-100 µg/ml) for 1h, followed by viability assessment by PI staining and flow cytometry. Combination index (CI) was calculated using CompuSyn software. Presented are median CI values. Statistical significance was determined using two-way ANOVA with Tukey's correction;

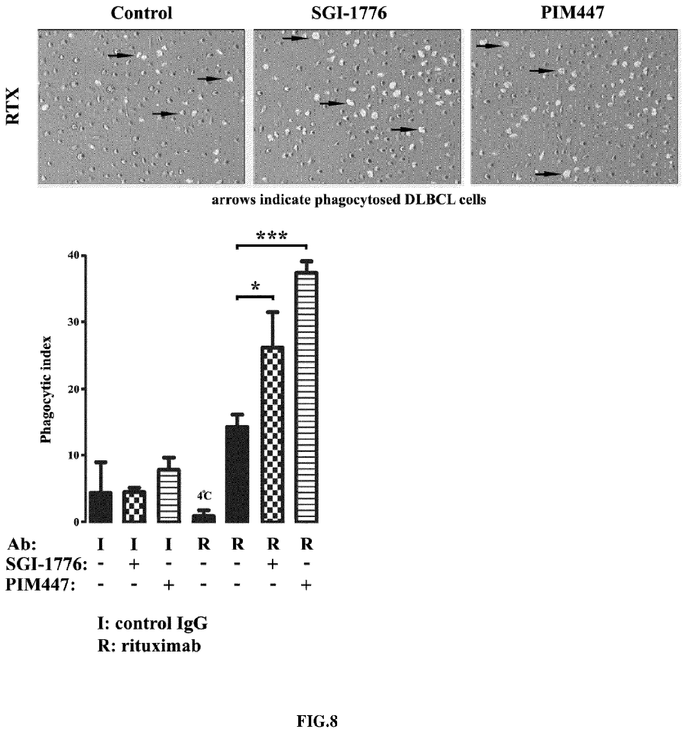

FIG. 8. shows that PIM inhibition in DLBCL cells potentiates rituximab-dependent phagocytosis. After 72 hours of treatment with DMSO, SIG-1776 (3µM) or PIM447 (7µM), CFSE-labeled DHL4 cells were incubated at 37°C with human peripheral blood-derived macrophages in the presence of IgG isotype control or rituximab (1µg/ml). As an additional control, to assure the specificity of the assay and exclude cell adhesion rather than phagocytosis as a source of colocalization of macrophages and DLBCL cells, DMSO-treated cells were incubated with macrophages and Rituximab (1µg/ml) at 4°C. A this low temperature, cellular metabolic activity and energy production are slowed/arrested and energy-demanding process of phagocytosis does not occur. Following incubation, cells were assessed by immunofluorescence microscopy for the presence of fluorescently-labeled DLBCL cells within the macrophages (indicated by arrows). Bar graphs present the mean phagocytic index, (the number of ingested CFSE-labelled DLBCL cells per 100 macrophages) of three independent replicates in a representative experiment, determined for each condition. P values were determined using the 2-sided Gosset's t-test: *P , .05; **P , .01; ***P , .001;

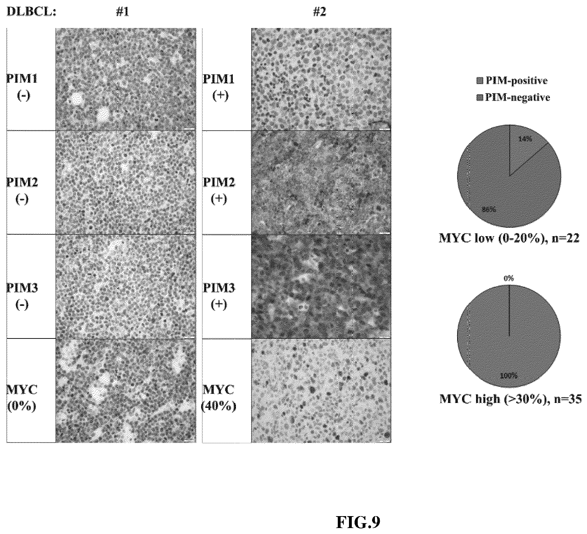

FIG. 9. Presents the expression of MYC and PIM kinases in DLBCL. Immunohistochemical analysis of MYC and PIM expression in DLBCL samples. Shown are representative cases, MYC-low and PIM1/2/3-negative (#1) and MYC-high and PIM1/2/3-positive (#2). Given values (%) refer to the percent of the malignant cells exhibiting MYC expression. Original magnification was 600x.

### DETAILED DESCRIPTION OF THE INVENTION

Without wishing to be bound to any theory, the inventors contemplate that the pan-PIM inhibitors (SGI-1776 and PIM447), and most likely also other compounds targeting PIM kinases, when used together with an anti-CD20 antibody, such as rituximab, synergistically induce death and potentiate phagocytosis of the malignant B-NHL cells expressing CD20 antigen, including, but not limited to DLBCL and BL cells.

Despite molecular and biological diversity of these diseases, the current frontline therapy for B-NHL patients usually consists of chemotherapy combined with anti-CD20 antibody - rituximab. Since approval, the antibody become a standard component of care for follicular lymphoma, diffuse large B-cell lymphoma, chronic lymphocytic leukemia, mantle cell lymphoma, and other B-cell tumors. For all of these diseases, clinical trials have demonstrated that rituximab not only prolongs the time to disease progression but also extends overall survival. However, rituximab exerts limited or no activity in B-NHL patients with decreased/low expression of CD20 antigen. Hence, strategies augmenting CD20 expression and thus activity of anti-CD20 antibodies are highly desired and are expected to improve treatment and extend lifespan of patients which belong to this unfavorable subgroup of B-NHLs. In this regard, the inventors indicate B-NHL patients would benefit from simultaneous, more specifically concurrent, or sequential administration of pan-PIM inhibitor to rituximab-based therapy through enhancing two major mechanisms of rituximab action, complement-dependent cytotoxicity of the antibody and antibody-dependent cellular phagocytosis.

The basis of present invention was the identification of yet unknown regulatory network in which PIM kinases support expression of MYC and MYC represses CD20 expression in B-NHL cells. Since MYC is regulated by multiple other kinases and regulatory proteins, and CD20 is regulated by many transcriptional modulators with potential compensating/complementing functions, it was not possible to predict prior to the experimental work described herein that PIM inhibition would synergize with anti-CD20 antibodies. The inventors characterized MYC-dependent mechanisms of CD20 repression, that include direct association of MYC with regulatory elements of MS4A1 gene and induction of miR-222, a microRNA identified by the inventors to target CD20. Consequently, inhibition of PIM kinases downregulates MYC and elevates surface CD20 abundance in B-NHL cells, making them more susceptible for rituximab-mediated toxicity.

To deliver a clinically applicable therapeutic modality working in synergy with CD20 antibodies, the inventors analyzed the regulation of MS4A1 transcription and sought for a targetable mechanism that would increase its expression, CD20 surface abundance, and efficacy of anti-CD20 antibody-based therapies. The inventors describe herein a PIM-MYC regulatory circuit that repress CD20 expression. They characterize PIM kinase inhibition as a strategy to modulate CD20 surface expression via blocking MYC activity. Finally, they demonstrate synergy between pan-PIM inhibitors and anti-CD20 antibodies in a preclinical model.

With respect to the clinically applicable dosing schedule in the newly diagnosed patients, PIM inhibitor should be given either prior to anti-CD20 antibody administered in standard does iv or sc, or simultaneously, more specifically concurrently, with the antibody.

PIM inhibition increases CD20 expression in lymphoma cells with a 1-3 day latency, so sequential dosing schedule would allow the cells to maximally induce CD20 prior to the antibody therapy. Moreover, due to the long anti-CD20 antibody half-life (median 3 weeks, range, 248-859 hours) (28) the synergistic effect is expected to be maintained even with simultaneous/concurrent dosing. This possibility of simultaneous or concurrent administration of PIM inhibitor as a first active ingredient and an anti-CD20 antibody as a second active ingredient to a patient in need thereof is irreplaceable for improving the reduction of the inconvenience of taking medications. Taking the above into account, it is worth emphasizing that the active ingredients can be administered to the patient at one time, which also eliminates the inconvenience associated with taking many drips, a long stay in a hospital facility or multiple visits. In patients relapsing post anti-CD20 therapy and exhibiting decreased CD20 surface expression, similar dosing schedules are applicable.

The present invention relates to methods and compositions for selection of previously untreated or relapsed patients with CD20-positive B lymphocyte-derived disorders, including, but not limited to, diffuse large B-cell lymphoma of ABC and GCB subtype according to WHO2016 classification of lymphoid tumors, or according to any other classifier, high-grade B-cell lymphoma, with MYC and BCL2 and/or BCL6 rearrangements, chronic lymphocytic leukemia, mantle cell lymphoma, Burkitt lymphoma, follicular lymphoma, or relapsed patients with low levels of CD20 surface expression who might be the candidates to boost its abundance prior the next line of therapy . In particular, the methods and compositions of the present invention relate to simultaneous, more precisely concurrent, or sequential use either isoform-specific or pan-PIM inhibitors, or multikinase inhibitors with PIM-blocking activity, with anti-CD20 targeting antibodies. The methods and compositions of the present invention can utilize PIM inhibitors to modulate expression and/or activity of gene products encoded by the MS4A1 gene (i.e., CD20 antigen).

### EXAMPLES

### METHODS

### Cell lines, culture conditions and chemicals

Human DLBCL cells (DHL4) and BL (RAJI) were maintained in RPMI 1640 medium supplemented with 10mM 4-(2-hydroxyethyl)-1-piperazineethanesulfonic acid (HEPES buffer), 50U/ml penicillin, 50U/ml streptomycin and 10% heat-inactivated FBS (Lonza). Macrophages were differentiated from peripheral blood CD14+ monocytes obtained from healthy volunteers. Briefly, mononuclear cells from buffy coats were isolated by density gradient centrifugation (Histopaque) and monocytes purified using CD14 magnetic microbeads (Miltenyi, cat. 130-050-201). To differentiate into macrophages, monocytes were cultured in TexMACS medium (Miltenyi, 130-097-196) supplemented with 50 U/mL penicillin, 50 U/mL streptomycin and 100ng/ml CSF-1 (R&D, cat. 216-MC-005) followed by 2-day culture period in CSF-1-deficient medium. If not otherwise specified, cells were grown in a humified atmosphere at 37°C with 5% CO2. The pan-PIM inhibitors SGI-1776 and PIM447 were purchased from Selleckchem and dissolved in DMSO. Rituximab (MabThera) was obtained from a commercial source.

### Immunoblotting

Cells were lysed in RIPA (150 mM NaCl, 1% NP40, 0,5% sodium deoxycholate, 0,1% SDS, 50 mM Tris HCl, pH=8) buffer containing protease inhibitor mixture (Complete; Roche Applied Science). Equal protein amounts were dissolved in sample buffer (4% SDS, 125 mM Tris/HCl (pH=6,8), 20% glycerol, 0,02% bromophenol blue, 10% β-merkaptoethanol) and were resolved by electrophoresis in polyacrylamide denaturing gels (SDS-PAGE - sodium dodecyl sulfate polyacrylamide gel electrophoresis) according to standard laboratory protocols described elsewhere (29). Electrophoresed proteins were electrotransferred to polyvinylidene difluoride (PVDF) membranes (Millipore) (29). Membranes were blocked with 5% non-fat milk dissolved in PBS-T (phosphate - buffered saline with 0.1%Tween-20) buffer for 1 h, and immunoblotted with primary and appropriate HRP-labelled secondary antibodies, diluted in PBS-T with 5% non-fat milk. Following primary antibodies were used: anti-MYC (abcam, ab32072, dilution 1:10000), anti-GAPDH (Merck, MAB374, dilution 1:2000) Signals were developed by enhanced luminescence using ECL reagent (Perkin Elmer) and a digital image acquisition system (G:Box, Syngene). Densitometric quantifications of band intensities were performed using ImageJ software (https://imagej.nih.gov/ij/).

### Flow cytometry

Surface CD20 expression was assessed using FITC-conjugated antibody from Beckman Coulter (A07772) and a control FITC-IgG2a isotype-matched antibody (BD 555573) as described before (22). Briefly, cells were stained cells with control IgG or CD20 antibodies, washed in PBS and analyzed using Cytoflex flow cytometer (Beckman Coulter). Expression data from 1x10⁴ cells were visualized in a single-parameter histograms displaying relative fluorescence of FITC on the x-axis (log scale) and cell count on the y-axis (linear scale). Viability of cells (% of apoptotic cells) was assessed by propidium iodide (PI) staining. Briefly, 1x10⁵ cells were stained with PI for 1 minute in the dark, followed by PI fluorescence assessment using Cytoflex instrument. Data were acquired from 1x10⁴ cells and % of PI-positive (apoptotic) cells determined from dot-plots of forward scatter versus PI.

### Real-time, quantitative polymerase chain reaction (qPCR)

RNA was extracted using a GeneMATRIX Universal RNA purification kit (EURx) and reverse-transcribed using Superscript III RT (Invitrogen), according to the manufacturer's instructions. MS4A1 expression levels, relative to RPL29 gene were measured in triplicates with the CFX RT-PCR system (BioRad) using the SYBR Green PCR Master Mix (BioRad) and the gene-specific primers (MS4A1-F: GAATGGGCTCTTCCACATTGCC, MS4A1-R: TCTCCGTTGCTGCCAGGAGT, RPL29-F: CAGCTCAGGCTCCCAAAC, RPL29-R: GCACCAGTCCTTCTGTCCTC. Obtained CT values for target genes and housekeeping control (RPL29) were used to calculate relative transcript abundance using 2^{-ΔΔCT} method. Detection and quantification of mature miRNAs was achieved by conducting reverse transcription quantitative real-time PCR (RT-qPCR) in conjunction with the TaqMan miRNA Assay (Applied Biosystems, cat. 4427975, assay ID 002276 and 001973) according to the manufacturer's protocol. Reverse transcription of RNA to cDNA was achieved by using the TaqMan MicroRNA Reverse Transcription Kit (Applied Biosystems, cat. cat 4366596) and qPCR using TaqMan Universal Master Mix II (cat. UNG 4440040). Changes in gene expression were calculated using 2^{-ΔΔCT} method, relative to U6 RNA expression.

*Chromatin immunoprecipitation (ChIP).*

ChIP experiments in DHL4 cells were performed using SimpleChIP Enzymatic Chromatin IP kit, according to protocol provided by manufacturer (Cell Signaling, cat. F9003S). Briefly, cells were fixed with formaldehyde followed by chromatin isolation and enzymatic shearing. Shared chromatin was immunoprecipitated with control IgG isotype antibody (Cell Signaling, cat. 5415S) or anti-Myc antibody (abcam, ab32072). Immunoprecipitated DNA was analysed by qPCR using SYBR Green PCR Master Mix (BioRad,) and MS4A1 promoter-specific primers: -313/-198-For: AGTGAAGCCAGAAGGTAAAAGT, -313/-198-Rev: GCTACCTTAATTAGAAGAACTTC, - 126/-39-For: GGTAAGGCAGAAACTTTTTTTGC ,-126/-39-Rev: CATCAGGTGACAGGAAATCAG. Obtained values were normalized to reference (input DNA).

### Complement-dependent cytotoxicity assay

DHL4 cells were treated with DMSO alone, 3µM SGI-1776 for 48h or 5µM PIM447 for 72h. RAJI cells were incubated with DMSO, 2µM SGI-1776 for 48h or 5µM PIM447 for 72h. Following treatment, 100000 cells per well were suspended in 200µl RPMI medium supplemented with 10% human serum (as a source of complement) and plated into 96-well plate. Cells were incubated with increasing concentrations of rituximab (0.1-100µg/ml) for 1 hour in a humified atmosphere at 37°C with 5% CO2. Afterwards, cell viability was assessed by PI staining and flow cytometry. Cell survival was calculated as a percentage of control cells incubated without antibody. ±SD. Statistical significance was determined using two-way ANOVA with Tukey's correction.

### Antibody-dependent cellular phagocytosis (ADCP) assay

To distinguish DLBCL cells from macrophages, DLBCL cells were labelled with CSFE and incubated for 3 days in the presence of pan-PIM inhibitors or DMSO as indicated. Thereafter, the cells were suspended in ice-cold and unsupplemented RPMI1640 medium at 1x10⁶/ml, and coated for 30 min with 1 µg/ml rituximab or IgG isotype-matched control antibody (Biolegend, 403501) at 4°C. Afterwards, the differentiated macrophages were washed with PBS and the rituximab-coated DLBCL cells were added to the wells at a 1:5 effector : target ratio. After 2h incubation at 37°C/5%CO2, free-floating, not phagocytosed cells were washed away with PBS and phagocytosis analyzed using Eclipse Ti2 fluorescent microscope (Nikon). The phagocytic index was determined by manual counting and calculated as the number of ingested CFSE-labelled DLBCL cells per 100 macrophages in three independent replicates in a representative experiment.

### Luciferase assay

3' Untranslated region (UTR) fragments of MS4A1 gene (CD20) containing the predicted miR-222-matching wild-type (WT: 5'-TCGAGTAGCTCCTTCTCTCTTACATTGAATGTAGAGAATGTAGCCATTGTAGCAGCTTG TGTTGTCACGCTTCTTCTTTTGAGCGC-3') and mutated (MUT: 5'-TCGAGTAGCTCCTTCTCTCTTACATTGAATGTAGAGAACGTTGCCATTGTAGCAGCTTG TGTTGTCACGCTTCTTCTTTTGAGCGC-3') sequence were synthesized by Sigma-Aldrich, annealed, and cloned into psiCheck-2 luciferase reporter vector (Promega). MiRIDIAN microRNA hsa-miR-222-3p mimic and miRIDIAN microRNA mimic negative control #1 were obtained from Invitrogen/ThermoScientific. HEK293T cells were transfected with psiCheck-2 control plasmid, psiCheck-2-CD20UTR-miR-222WT or psiCheck-2-CD20UTR-miR-222MUT vector and an appropriate miRNA mimic using Lipofectamine 2000 (Thermo Fisher). Twenty-four hours after transfection, luciferase activities were determined as described previously (30).

### RNAi-mediated silencing of gene expression

SiRNAs targeting PIM1 (cat. 4390824, Ambion), PIM2 (cat. L-005359-00, Dharmacon), PIM3 (cat. PIM3HSS140560, Invitrogen), MYC (cat. L-003282-02, Dharmacon) and control siRNA (cat. AM4611, Ambion) were delivered into DHL4 and RAJI cells by electroporation using Lonza Nucleofector 4D system and Invitrogen Neon device respectively. Electroporated cells were incubated at 37°C with 5% CO2 in RPMI medium supplemented with 5% FBS and knock-down efficiencies were determined 48 hours later by immunoblot.

### EXAMPLE 1

### MYC regulates CD20 expression in DLBCL and BL cells

To characterize the potentially targetable mechanism regulating CD20 expression in lymphoma cells, which might be clinically used to boost cellular CD20 levels, the inventors first analyzed promoter region of MS4A1 gene (encoding CD20), searching for transcription factor binding sites with a regulatory potential. These analyses revealed binding sites for previously characterized inducers and repressors of CD20 (Figure 4A). In addition, the inventors identified two binding sites for MYC transcription factor at positions -245/-240 and -45/-40 from the MS4A1transcription initiation site (Figure 4A). MYC may repress or induce transcription by binding to its cognate E-box elements, localized within promoter regions (31). To determine whether MYC binds to MS4A1 promoter directly in DLBCL cells, the inventors performed chromatin immunoprecipitation assay using control IgG antibody (control) or specific antibody against MYC and primers amplifying MS4A1 promoter regions of interest. Anti-MYC antibody immunoprecipitated higher amount of both MS4A1 promoter fragments that did the control antibody, confirming that MYC specifically binds to MS4A1 promoter at these sites (Figure 4B).

To elucidate the role of MYC in CD20 regulation in human DLBCL and BL cells, the inventors investigated MS4A1 mRNA levels in a publicly available microarray dataset (Gene Expression Omnibus (GEO) accession number GSE4475) encompassing 182 BL and DLBCL patients (32). Since MYC translocation to immunoglobulin locus is associated with elevated MYC protein levels and activity (5), the inventors divided BL and DLBCL patients into two groups, harboring wild-type (WT) or Ig-translocated (Tx) MYC. As depicted in Figure 5A, MS4A1 mRNA levels were significantly higher in MYC-WT patients than in those with MYC-Tx, suggesting that MYC represses CD20 in DLBCL and BL. To confirm the role of MYC in CD20 regulation, the inventors next investigated surface CD20 abundance in DHL4 DLBCL and RAJI BL cells by flow cytometry following siRNA-mediated MYC knockdown (Figure 5B). Genetic MYC depletion was associated with increased surface CD20 levels in both DHL4 and RAJI cells (Figure 5C). In line with decreased MS4A1 mRNA levels observed in patients harboring MYC translocations (Figure 5A), MYC knockdown increased MS4A1 mRNA abundance in both investigated cell lines (Figure 5D). Collectively, these data indicate that MYC downregulates transcript levels of MS4A1.

Since MYC is a potent modulator of transcription of numerous micro RNAs (miRNAs) (33), the inventors sought for additional, miRNA-driven, indirect, MYC- dependent mechanisms regulating CD20 expression. At first, the inventors screened a curated list of known 59 MYC-upregulated miRNAs for potential interactions with MS4A1 transcript using TargetScan algorithm (34). Using this approach, the inventors identified miR-222 matching sequences in 3'UTR of MS4A1 gene. To investigate whether miR-222 targets and represses MS4A1 directly through 3'UTR interaction, the inventors cloned 3'UTR fragments of this gene into a luciferase reporter plasmid. The inventors observed significant decrease in luciferase activity in cells cotransfected with vector containing wild-type matching sequences from 3'UTR of MS4A1 gene and miR-222 mimic (Figure 5E). In contrast, luciferase activities from reporter vectors carrying 3'UTRs with mutated seed sequences for miR-222 were not decreased, confirming the specificity of these interactions.

To assess role of miR-222 in repression of MS4A1 in lymphoma cells, the inventors investigated mRNA and protein abundance of CD20 in DHL4 cells transfected with miR-222 mimic or a control non-targeting miR mimic. As expected, miR-222 overexpression decreased mRNA and surface CD20 expression (Figure 5F-G). To assess miR-222 dependence on MYC, DHL4 cells were incubated with MYC inhibitor 10058-F4 and miR-222 expression was assessed by qPCR. In line with previous findings (34), MYC inhibition in DLBCL cells was associated with markedly decreased levels of miR-222 (Figure 5H).

Taken together, data presented by the inventors indicate that MYC downregulates CD20 expression in B-NHL cells by two mechanisms: by direct binding to MS4A1 promoter and indirectly, by upregulation of miRNA-222.

### EXAMPLE 2

### Inhibition of PIM kinases in B-NHL cells downregulates MYC and elevates CD20 expression

Given earlier observations (26,27) and the inventor's data indicating that MYC negatively regulates CD20 expression in B-NHL cells, the inventors hypothesized that PIM expression contributes to CD20 repression by increasing MYC abundance. However, since MYC is regulated by multiple other kinases and regulatory proteins, and CD20 is regulated by many transcriptional modulators with potential compensating/complementing functions, it was not possible to precisely predict the effect of PIM inhibition on CD20 expression. To test this hypothesis, the inventors investigated MYC and CD20 expression levels in DHL4 and RAJI cells following genetic ablation and pharmacological inhibition of all three PIM kinases. Genetic knockdown of PIM kinases in B-NHL cells was associated marked reduction of MYC protein abundance (Figure 6A). Importantly, PIM-knockdown cells also exhibited increased surface levels of CD20 compared to PIM-expressing control cells (Figure 6B). These observations were recapitulated using commercially available pan-PIM inhibitors, SGI-1776 and PIM447 (Figure 6C-D). Collectively, inventor's data demonstrate that PIM kinase inhibition downregulates MYC and consequently induces CD20 expression in B-NHL cells.

### EXAMPLE 3

### PIM kinase inhibitors potentiate anti-CD20 antibody activity in B-NHL cells

Since pharmacological PIM kinase blockade led to elevated CD20 levels, the inventors next asked whether pan-PIM inhibitors would potentiate CDC of an anti-CD20 antibody in B-NHL cells. To answer this question, DMSO-, SGI-1776 or PIM447-treated DHL4 and RAJI cells were incubated for 1 hour with increasing doses of rituximab (0.1-100 µg/ml), in the presence of 10% human serum as a source of complement. Following incubation, cells were stained with propidium iodide (PI) and % of PI-positive (apoptotic) cells assessed by flow cytometry. In comparison to DMSO-treated, control cells exposed to rituximab, DHL4 and RAJI cells incubated with both SGI-1776 and PIM447 exhibited marked increase in apoptosis (Figure 7). Importantly, calculated combinatory indexes (CI) revealed synergistic effects of rituximab in conjunction with each of the pan-PIM inhibitor tested.

Since antibody-dependent cellular phagocytosis (ADCP) of tumor cells by macrophages represents another important mechanism of anti-CD20 monoclonal antibody action in B-NHLs, the inventors next explored the effects of PIM inhibition on ADCP. For this purpose, the inventors co-incubated CFSE-labelled, DMSO- or SGI-1776- or PIM447-treated DHL4 cells with human blood-derived macrophages at a 1:5 effector : target ratio for 2h at 37°C/5%CO2 , in the presence of IgG isotype control or rituximab (1µg/ml), and assessed number of CSFE-labelled DLBCL cells ingested by macrophages, by immunofluorescence microscopy. As depicted in Figure 8, treatment with pan-PIM inhibitors potentiated rituximab-dependent phagocytosis of the investigated DLBCL cells. In addition, to assure the specificity of the assay and exclude cell adhesion rather than phagocytosis as a source of colocalization of macrophages and DLBCL cells, we conducted the assay at 4°C. A this low temperature, cellular metabolic activity and energy production are slowed/arrested and energy-demanding process of phagocytosis does not occur.

Collectively, these results demonstrate that PIM inhibition increases CD20 levels and PIM inhibitors synergize with anti-CD20 antibody-based therapies in B-NHLs.

### EXAMPLE 4

To identify patients that would respond to the combined treatment with pan-PIM inhibitors and rituximab, the inventors assessed expression of MYC and PIM kinases in a group of 57 patients diagnosed with DLBCL, using immunohistochemistry. Unexpectedly, the inventors found that expression of PIM kinases characterizes all patients exhibiting high MYC levels, whereas most of patients (86%) with low MYC abundance were PIM-negative (Figure 9). Given that and the disclosed by the inventors PIM-MYC circuit regulating CD20 expression in B-NHL cells, only patients exhibiting high MYC expression levels, thus co-expressing MYC and PIM kinases, would likely benefit from combinatory pan-PIM inhibitor and rituximab treatment. Hence, immunohistochemical MYC assessment represents potential a biomarker of combined activity of PIM kinase inhibitors and rituximab in DLBCL.

The regimen of administration can affect what constitutes an effective amount.

The inhibitor compound can be administered to the subject either prior to or after the onset of cancer. Further, several divided dosages, as well as staggered dosages, can be administered daily or sequentially, or the dose can be continuously infused, or can be a bolus injection. Further, the dosages of the PIM inhibitor(s) can be proportionally increased or decreased as indicated by the exigencies of the therapeutic or prophylactic situation.

PIM inhibitor should be given either prior to anti-CD20 antibody administered intravenously or subcutaneously, or simultaneously, more specifically concurrently, with the antibody. PIM inhibitors can be given either orally, subcutaneously, intravenously or via any other route of administration, depending on the inhibitor compound pharmacokinetics and bioavailability. PIM inhibition increases CD20 expression in lymphoma cells with a 1-3 day latency, so sequential dosing schedule would allow the cells to maximally induce CD20 prior to the antibody therapy. Given long anti-CD20 antibody half-life (median 3 weeks, range, 248-859 hours) (28), simultaneous dosing is also justifiable. In patients relapsing post anti-CD20 therapy and exhibiting decreased CD20 surface expression, similar dosing schedules are applicable.

### REFERENCES

1. Kubuschok B, Held G, Pfreundschuh M. Management of diffuse large B-cell lymphoma (DLBCL). Cancer Treat Res. 2015;165:271-288.
2. Chapuy B, Stewart C, Dunford AJ, et al. Author Correction: Molecular subtypes of diffuse large B cell lymphoma are associated with distinct pathogenic mechanisms and outcomes. Nat Med. 2018;24(8):1290-1291.
3. Li L, Li Y, Que X, et al. Prognostic significances of overexpression MYC and/or BCL2 in R-CHOP-treated diffuse large B-cell lymphoma: A Systematic review and meta-analysis. Sci Rep. 2018;8(1):6267.
4. Copie-Bergman C, Cuilliere-Dartigues P, Baia M, et al. MYC-IG rearrangements are negative predictors of survival in DLBCL patients treated with immunochemotherapy: a GELA/LYSA study. Blood. 2015;126(22):2466-2474.
5. Sewastianik T, Prochorec-Sobieszek M, Chapuy B, Juszczynski P. MYC deregulation in lymphoid tumors: molecular mechanisms, clinical consequences and therapeutic implications. Biochim Biophys Acta. 2014;1846(2):457-467.
6. Moroy T, Verbeek S, Ma A, Achacoso P, Berns A, Alt F. E mu N- and E mu L-myc cooperate with E mu pim-1 to generate lymphoid tumors at high frequency in double-transgenic mice. Oncogene. 1991;6(11):1941-1948.
7. Alvarado Y, Giles FJ, Swords RT. The PIM kinases in hematological cancers. Expert Rev Hematol. 2012;5(1):81-96.
8. Huang SM, Wang A, Greco R, et al. Combination of PIM and JAK2 inhibitors synergistically suppresses MPN cell proliferation and overcomes drug resistance. Oncotarget. 2014;5(10):3362-3374.
9. Lu J, Zavorotinskaya T, Dai Y, et al. Pim2 is required for maintaining multiple myeloma cell growth through modulating TSC2 phosphorylation. Blood. 2013;122(9):1610-1620.
10. Chen LS, Redkar S, Bearss D, Wierda WG, Gandhi V. Pim kinase inhibitor, SGI-1776, induces apoptosis in chronic lymphocytic leukemia cells. Blood. 2009;114(19):4150-4157.
11. Chen LS, Redkar S, Taverna P, Cortes JE, Gandhi V. Mechanisms of cytotoxicity to Pim kinase inhibitor, SGI-1776, in acute myeloid leukemia. Blood. 2011;118(3):693-702.
12. Gomez-Abad C, Pisonero H, Blanco-Aparicio C, et al. PIM2 inhibition as a rational therapeutic approach in B-cell lymphoma. Blood. 2011;118(20):5517-5527.
13. Coiffier B, Thieblemont C, Van Den Neste E, et al. Long-term outcome of patients in the LNH-98.5 trial, the first randomized study comparing rituximab-CHOP to standard CHOP chemotherapy in DLBCL patients: a study by the Groupe d'Etudes des Lymphomes de I'Adulte. Blood. 2010;116(12):2040-2045.
14. Horvat M, Kloboves Prevodnik V, Lavrencak J, Jezersek Novakovic B. Predictive significance of the cut-off value of CD20 expression in patients with B-cell lymphoma. Oncol Rep. 2010;24(4):1101-1107.
15. Hiraga J, Tomita A, Sugimoto T, et al. Down-regulation of CD20 expression in B-cell lymphoma cells after treatment with rituximab-containing combination chemotherapies: its prevalence and clinical significance. Blood. 2009;113(20):4885-4893.
16. Williams ME, Densmore JJ, Pawluczkowycz AW, et al. Thrice-weekly low-dose rituximab decreases CD20 loss via shaving and promotes enhanced targeting in chronic lymphocytic leukemia. J Immunol. 2006;177(10):7435-7443.
17. Johnson NA, Boyle M, Bashashati A, et al. Diffuse large B-cell lymphoma: reduced CD20 expression is associated with an inferior survival. Blood. 2009;113(16):3773-3780.
18. Czuczman MS, Olejniczak S, Gowda A, et al. Acquirement of rituximab resistance in lymphoma cell lines is associated with both global CD20 gene and protein down-regulation regulated at the pretranscriptional and posttranscriptional levels. Clin Cancer Res. 2008;14(5):1561-1570.
19. Small GW, McLeod HL, Richards KL. Analysis of innate and acquired resistance to anti-CD20 antibodies in malignant and nonmalignant B cells. PeerJ. 2013;1:e31.
20. Bojarczuk K, Siernicka M, Dwojak M, et al. B-cell receptor pathway inhibitors affect CD20 levels and impair antitumor activity of anti-CD20 monoclonal antibodies. Leukemia. 2014;28(5):1163-1167.
21. Da Roit F, Engelberts PJ, Taylor RP, et al. Ibrutinib interferes with the cell-mediated anti-tumor activities of therapeutic CD20 antibodies: implications for combination therapy. Haematologica. 2015;100(1):77-86.
22. Pyrzynska B, Dwojak M, Zerrouqi A, et al. FOXO1 promotes resistance of non-Hodgkin lymphomas to anti-CD20-based therapy. Oncoimmunology. 2018;7(5):e1423183.
23. Winiarska M, Bojarczuk K, Pyrzynska B, et al. Inhibitors of SRC kinases impair antitumor activity of anti-CD20 monoclonal antibodies. MAbs. 2014;6(5):1300-1313.
24. Morishita D, Katayama R, Sekimizu K, Tsuruo T, Fujita N. Pim kinases promote cell cycle progression by phosphorylating and down-regulating p27Kip1 at the transcriptional and posttranscriptional levels. Cancer Res. 2008;68(13):5076-5085.
25. Xu Z, Gwin KA, Li Y, Medina KL. Developmental stage-specific effects of Pim-1 dysregulation on murine bone marrow B cell development. BMC Immunol. 2016;17(1):16.
26. Yu D, Dews M, Park A, Tobias JW, Thomas-Tikhonenko A. Inactivation of Myc in murine two-hit B lymphomas causes dormancy with elevated levels of interleukin 10 receptor and CD20: implications for adjuvant therapies. Cancer Res. 2005;65(12):5454-5461.
27. Seitz V, Butzhammer P, Hirsch B, et al. Deep sequencing of MYC DNA-binding sites in Burkitt lymphoma. PLoS One. 2011;6(11):e26837.
28. Regazzi MB, Iacona I, Avanzini MA, et al. Pharmacokinetic behavior of rituximab: a study of different schedules of administration for heterogeneous clinical settings. Ther Drug Monit. 2005;27(6):785-792.
29. Sambrook et al., Molecular Cloning, A Labolatory Manual. 1989, New York: Cold Spring Harbor Labolatory Press
30. Sewastianik T, Szydlowski M, Jablonska E, et al. FOXO1 is a TXN- and p300-dependent sensor and effector of oxidative stress in diffuse large B-cell lymphomas characterized by increased oxidative metabolism. Oncogene. 2016;35(46):5989-6000.
31. Satou A, Taira T, Iguchi-Ariga SM, Ariga H. A novel transrepression pathway of c-Myc. Recruitment of a transcriptional corepressor complex to c-Myc by MM-1, a c-Myc-binding protein. J Biol Chem. 2001;276(49):46562-46567.
32. Hummel M, Bentink S, Berger H, et al. A biologic definition of Burkitt's lymphoma from transcriptional and genomic profiling. N Engl J Med. 2006;354(23):2419-2430.
33. Jackstadt R, Hermeking H. MicroRNAs as regulators and mediators of c-MYC function. Biochim Biophys Acta. 2015;1849(5):544-553.
34. Kim JW, Mori S, Nevins JR. Myc-induced microRNAs integrate Myc-mediated cell proliferation and cell fate. Cancer Res. 2010;70(12):4820-4828.

## Claims

1. The PIM inhibitor for use in the treatment of hematological malignancies in combination therapy with anti-CD21 antibody wherein said PIM kinase inhibitor is selected from the group comprising SGI-1776, PIM447 and SEL24/MEN1703.

2. The PIM inhibitor for use according to claim 1, wherein said hematological malignancy is selected from a group consisting of diffuse large B-cell lymphoma, high-grade B-cell lymphoma with MYC and BCL2 and/or BCL6 rearrangements, chronic lymphocytic leukemia/lymphocytic lymphoma, mantle cell lymphoma, Burkitt lymphoma, follicular lymphoma, marginal zone lymphoma.

3. The PIM inhibitor for use according to claim 1 or 2, wherein the treatment comprises administering to the patient a therapeutically effective amount of the PIM kinase inhibitor and a therapeutically effective amount of anti-CD20 antibody.

4. The PIM inhibitor for use according to any of claims 1-3, wherein the administration of the PIM kinase inhibitor and the anti-CD20 antibody is performed simultaneously, more specifically concurrently.

5. The PIM inhibitor for use according to any one of the claims 1-3 wherein the PIM kinase inhibitor and the anti-CD20 antibody are administered sequentially.

6. The PIM inhibitor for use according to claim 5 wherein the administration of first dose of the PIM kinase inhibitor is performed before administration of first dose of anti-CD20 antibody, preferably by at least 12 hours before administration of the first dosage of anti-CD20 antibody, more preferably by at least 24 or 48 hours before administration of the first dose of anti-CD20 antibody, most preferably by at least 24 hours before administration of the first dose of anti-CD20 antibody.

7. The PIM inhibitor for use according to any one of the preceding claims, wherein the administration of the PIM kinase inhibitor and anti-CD20 antibody is performed in therapeutic cycles, preferably said therapeutic cycles include dosing of the PIM kinase inhibitor for 14 subsequent days starting from day 1 of the therapeutic cycle and dosing of the anti-CD20 antibody for 21 subsequent days starting from day 2 of the therapeutic cycle, wherein the anti-CD20 antibody is administered with at least a 24 hours delay with respect to beginning of the PIM kinase inhibitor administration.

8. The PIM inhibitor for use according to any one of the preceding claims, wherein the administration of the PIM inhibitor and the anti-CD20 antibody is performed simultaneously, more specifically concurrently, with an additional therapy, preferably with chemotherapy, more preferably with R-CHOP chemotherapy.

9. A combination therapy pharmaceutical composition comprising an effective amounts of the PIM inhibitor and an anti-CD20 antibody as active ingredients, and pharmaceutically acceptable excipients, wherein said PIM kinase inhibitor is selected from the group comprising SGI-1776, PIM447 and SEL24/MEN1703.

10. The combination therapy pharmaceutical composition according to claim 9, wherein the active ingredients are formulated in separate dosage formulations, preferably such separate dosage formulations are intended to be administered simultaneously, more specifically concurrently, or under different dosage regimes.

11. The combination therapy pharmaceutical composition according to any of claims 9 - 10 for use in the treatment of hematologic malignancy.

## Patentansprüche

1. Das PIM-Inhibitor zur Anwendung bei der Behandlung von hämatologischen Malignomen in Kombinationstherapie mit einem Anti-CD20-Antikörper,
wobei der PIM-Kinaseinhibitor aus einer Gruppe ausgewählt ist, die SGI-1776, PIM447 und SEL24/MEN1703 umfasst.

2. Das PIM-Inhibitor zur Anwendung gemäß Anspruch 1, wobei die erwähnte maligne hämatologische Erkrankung aus einer Gruppe ausgewählt ist, die umfasst: diffuses großzelliges B-Zell-Lymphom, hochgradiges B-Zell-Lymphom mit MYC- und BCL2- und/oder BCL6-Rearrangements, chronische lymphatische Leukämie/ lymphozytäres Lymphom, Mantelzell-Lymphom, Burkitt-Lymphom, follikuläres Lymphom und Marginalzonen-Lymphom.

3. Das PIM-Inhibitor zur Anwendung nach Anspruch 1 oder 2, wobei die Therapie in der Verabreichung einer therapeutisch wirksamen Menge des PIM-Kinaseinhibitors und einer therapeutisch wirksamen Menge des Anti-CD20-Antikörpers an den Patienten besteht.

4. Das PIM-Inhibitor zur Anwendung nach einem der Ansprüche 1 - 3, wobei die Verabreichung des PIM-Kinaseinhibitors und des Anti-CD20-Antikörpers gleichzeitig, insbesondere spezifisch gemeinsam, erfolgt.

5. Das PIM-Inhibitor zur Anwendung nach einem der Ansprüche 1 - 3, wobei der PIM-Kinaseinhibitor und der Anti-CD20-Antikörper nacheinander verabreicht werden.

6. Das PIM-Inhibitor zur Anwendung nach Anspruch 5, wobei die Verabreichung der ersten Dosis des PIM-Kinaseinhibitors vor der Verabreichung der ersten Dosis des Anti-CD20-Antikörpers erfolgt, bevorzugt mindestens 12 Stunden vor der Verabreichung der ersten Dosis des Anti-CD20-Antikörpers, noch bevorzugter mindestens 24 oder 48 Stunden vor der Verabreichung der ersten Dosis des Anti-CD20-Antikörpers, am meisten bevorzugt mindestens 24 Stunden vor der Verabreichung der ersten Dosis des Anti-CD20-Antikörpers.

7. Das PIM-Inhibitor zur Anwendung nach einem der vorgenannten Ansprüche, wobei die Verabreichung des PIM-Kinaseinhibitors und des Anti-CD20-Antikörpers in therapeutischen Zyklen erfolgt, wobei die erwähnten therapeutischen Zyklen bevorzugt die Verabreichung des PIM-Kinaseinhibitors an 14 aufeinanderfolgenden Tagen, beginnend mit dem 1. Tag des therapeutischen Zyklus, und die Verabreichung des Anti-CD20-Antikörpers an 21 aufeinanderfolgenden Tagen, beginnend mit dem 2. Tag des therapeutischen Zyklus, umfassen, wobei der Anti-CD20-Antikörper mit einer Verzögerung von mindestens 24 Stunden in Bezug auf den Anfang der Verabreichung des PIM-Kinaseinhibitors verabreicht wird.

8. Das PIM-Inhibitor zur Anwendung nach einem der vorgenannten Ansprüche, wobei die Verabreichung des PIM-Inhibitors und des Anti-CD20-Antikörpers gleichzeitig, noch spezifischer gemeinsam, in Begleitung einer weiteren Therapie, bevorzugt der Chemotherapie, bevorzugt der R-CHOP-Chemotherapie, erfolgt.

9. Ein pharmazeutisches Gemisch für die Kombinationstherapie, umfassend wirksame Mengen des PIM-Inhibitors und des Anti-CD20-Antikörpers als Wirkstoffe sowie pharmazeutisch zulässige Hilfsstoffe, wobei der PIM-Kinase-Inhibitor aus einer Gruppe ausgewählt ist, die SGI-1776, PIM447 und SEL24/MEN1703 umfasst.

10. Das pharmazeutische Gemisch für die Kombinationstherapie nach Anspruch 9, wobei die Wirkstoffe in getrennten Dosierungsformulierungen formuliert sind, wobei diese getrennten Dosierungsformulierungen bevorzugt dazu bestimmt sind, gleichzeitig, spezifischer gemeinsam, oder nach verschiedenen Dosierungsschemata verabreicht zu werden.

11. Das pharmazeutische Gemisch für die Kombinationstherapie nach einem der Ansprüche 9 - 10 zur Anwendung bei der Behandlung von hämatologischem Malignom.

## Revendications

1. Inhibiteur de PIM pour son utilisation dans le traitement des hémopathies malignes en combinaison avec un anticorps anti-CD20
dans lequel ledit inhibiteur de la kinase PIM est choisi dans le groupe comprenant SGI-1776, PIM447 et SEL24/MEN1703.

2. Inhibiteur de PIM utilisé selon la revendication 1, dans lequel ladite tumeur maligne hématologique est choisie dans un groupe comprenant le lymphome diffus à grandes cellules B, le lymphome à cellules B de haut grade avec réarrangements MYC et BCL2 et/ou BCL6, la leucémie lymphocytaire chronique/le lymphome lymphocytaire, le lymphome à cellules du manteau, le lymphome de Burkitt, le lymphome folliculaire, le lymphome de la zone marginale.

3. Inhibiteur PIM à utiliser selon la revendication 1 ou 2, dans lequel le traitement comprend l'administration au patient d'une quantité thérapeutiquement efficace de l'inhibiteur de la kinase PIM et d'une quantité thérapeutiquement efficace d'anticorps anti-CD20.

4. Inhibiteur PIM utilisé selon l'une des revendications 1 à 3, dans lequel l'administration de l'inhibiteur de la kinase PIM et de l'anticorps anti-CD20 est effectuée simultanément, plus précisément de manière concomitante.

5. Inhibiteur PIM utilisé selon l'une des revendications 1 à 3, dans lequel l'inhibiteur de la kinase PIM et l'anticorps anti-CD20 sont administrés de manière séquentielle.

6. Inhibiteur de PIM utilisé selon la revendication 5, dans lequel l'administration de la première dose de l'inhibiteur de kinase PIM est effectuée avant l'administration de la première dose d'anticorps anti-CD20, de préférence au moins 12 heures avant l'administration de la première dose d'anticorps anti-CD20, plus préférablement au moins 24 ou 48 heures avant l'administration de la première dose d'anticorps anti-CD20, le plus préférablementau moins 24 heures avant l'administration de la première dose d'anticorps anti-CD20.

7. Inhibiteur de PIM utilisé selon l'une des revendications précédentes, dans lequel l'administration de l'inhibiteur de PIM kinase et de l'anticorps anti-CD20 est effectuée par cycles thérapeutiques, de préférence lesdits cycles thérapeutiques comprennent l'administration de l'inhibiteur de PIM kinase pendant 14 jours consécutifs à partir du jour 1 du cycle thérapeutique et l'administration de l'anticorps anti-CD20 pendant 21 jours consécutifs à partir du jour 2 du cycle thérapeutique, l'anticorps anti-CD20 étant administré avec un retard d'au moins 24 heures par rapport au début de l'administration de l'inhibiteur de PIM kinase.

8. Inhibiteur de PIM utilisé selon l'une des revendications précédentes, dans lequel l'administration de l'inhibiteur de PIM et de l'anticorps anti-CD20 est effectuée simultanément, plus précisément concurremment, avec une thérapie supplémentaire, de préférence avec une chimiothérapie, de préférence avec une chimiothérapie R-CHOP.

9. Composition pharmaceutique de thérapie combinée comprenant des quantités efficaces d'inhibiteur PIM et d'anticorps anti-CD20 en tant qu'ingrédients actifs, et des excipients pharmaceutiquement acceptables, dans laquelle ledit inhibiteur de kinase PIM est choisi dans le groupe comprenant SGI-1776, PIM447 et SEL24/MEN1703.

10. Composition pharmaceutique de thérapie combinée selon la revendication 9, dans laquelle les ingrédients actifs sont formulés dans des formulations de dosage séparées, de préférence ces préparations de dosage séparées sont destinées à être administrées simultanément, plus spécifiquement concurremment, ou selon des régimes de dosage différents.

11. Composition pharmaceutique de thérapie combinée selon l'une des revendications 9 à 10 pour le traitement d'une tumeur hématologique maligne.
